(19) Europäisches Patentamt
European Patent Office
Office européen des brevets

(11) EP 4 089 088 A1

(12) # EUROPEAN PATENT APPLICATION
published in accordance with Art. 153(4) EPC

(43) Date of publication:
**16.11.2022 Bulletin 2022/46**

(21) Application number: **21855433.5**

(22) Date of filing: **05.08.2021**

(51) International Patent Classification (IPC):
***C07D 405/14*** (2006.01) ***C07D 407/12*** (2006.01)
***C07D 409/14*** (2006.01) ***A61K 31/352*** (2006.01)
***A61K 31/4433*** (2006.01) ***A61K 31/4436*** (2006.01)
***A61P 29/00*** (2006.01)

(52) Cooperative Patent Classification (CPC):
**A61K 31/352; A61K 31/4433; A61K 31/4436; A61P 29/00; C07D 405/14; C07D 407/12; C07D 409/14**

(86) International application number:
**PCT/CN2021/110702**

(87) International publication number:
**WO 2022/033380 (17.02.2022 Gazette 2022/07)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **10.08.2020 CN 202010794491**

(71) Applicant: **Chengdu Easton Biopharmaceuticals Co., Ltd.**
**Hi-tech District**
**Chengdu, Sichuan 611731 (CN)**

(72) Inventors:
- **XIANG, Yongzhe**
**Chengdu, Sichuan 611731 (CN)**
- **ZENG, Yanqun**
**Chengdu, Sichuan 611731 (CN)**
- **ZHOU, Ning**
**Chengdu, Sichuan 611731 (CN)**
- **WANG, Ying**
**Chengdu, Sichuan 611731 (CN)**

(74) Representative: **Gulde & Partner**
**Patent- und Rechtsanwaltskanzlei mbB**
**Wallstraße 58/59**
**10179 Berlin (DE)**

(54) **MOR RECEPTOR AGONIST COMPOUND, PREPARATION METHOD THEREFOR, AND USE THEREOF**

(57) The present invention relates to the technical of medicinal chemistry, and specifically relates to a use of a 6-oxaspiro[4.5]decane derivative as an MOR receptor agonist for treating pain and pain-related disorders.

EP 4 089 088 A1

## Description

### TECHNICAL FIELD

The present invention relates to the field of medicinal chemistry, and specifically relates to use of a class of 6-oxaspiro[4.5]decane derivatives as MOR receptor agonists for treating pain and pain-related disorders.

### BACKGROUND

To date, opioid drugs, represented by morphine, are still the effective drugs mainly for the treatment of severe pain. These drugs have good analgesic activity, but their clinical application is greatly limited due to severe adverse reactions of the central nervous system, especially respiratory depression, constipation, tolerance and addiction and other side effects. For this reason, people have been dedicated to seeking the ideal opioid drug with good analgesic effect and less addiction, and during the process of continuous exploration of the structure-activity relationship of opioid drugs, people also have a deeper understanding of the *in vivo* specific material basis of their actions.

Studies have shown that there are three molecularly and pharmacologically distinct types of opioid receptors (OR): δ, κ and μ. Opioid drugs are primarily transferred through opioid μ receptor. The μ receptor is classic GPCRs, and a large number of studies have shown that the biased agonist of the μ receptor has better analgesic effect and can reduce the related side effects. This opens up the possibility of developing an ideal opioid drug. At present, the most advanced is TRV130 (oliceridine) from Trevena. In February 2017, TRV130 was partially successful in two Phase III clinical trials, with better postoperative analgesic effect than placebo, reaching the primary endpoint of the trial. However, in terms of improving tolerance, it does not show an apparent trend of superiority over morphine as expected.

Therefore, on the basis of existing domestic and foreign researches, the present invention strives to develop a μ-receptor biased agonist with better efficacy, longer duration of analgesia and lower side effects, providing a better choice for clinical application.

### SUMMARY OF THE INVENTION

The present invention relates to a class of 6-oxaspiro[4.5]decane derivatives as agonists for μ receptor, and specifically relates to a 6-oxaspiro[4.5]decane derivative, a preparation method thereof, and medical applications thereof, especially the 6-oxaspiro[4.5]decane derivatives represented by the following general formula (I) and use thereof in the preparation of analgesic drugs, more specifically, in the preparation of drugs suitable for moderate to severe pain.

One object of the present invention is to provide a 6-oxaspiro[4.5]decane derivative having a structure represented by the following general formula (I):

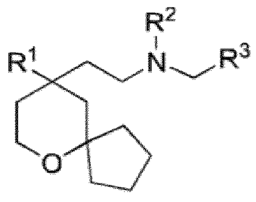

Formula I

or its tautomer, mesomer, racemate, enantiomer, diastereomer or mixture form thereof;
wherein,

- $R^1$ is selected from a phenyl group or a pyridyl group, wherein hydrogen atom(s) of the phenyl group or the pyridyl group can be further substituted by a fluorine atom or a chlorine atom;
- $R^2$ is selected from a hydrogen atom or a $C_1$-$C_3$ alkyl group;
- $R^3$ is selected from

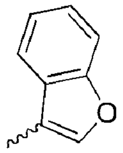 , 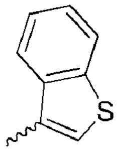 , or 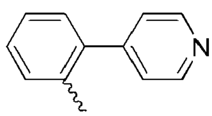 ,

and hydrogen atom(s) of the aryl group therein can be further substituted by a fluorine atom, a chlorine atom, an alkyl group, a heterocyclic group, or an alkoxy group, and the alkoxy group can be further substituted by either group selected from a heterocyclic group and an alkoxy group.

Preferably, in the compound represented by the general formula (I) of the present invention:

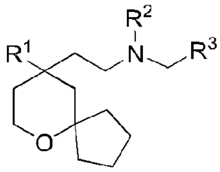

Formula I

$R^1$ is selected from a phenyl group or a pyridyl group, wherein hydrogen atom(s) of the phenyl group or the pyridyl group can be further substituted by a fluorine atom or a chlorine atom;
$R^2$ is selected from a hydrogen atom, a methyl group or an ethyl group;
$R^3$ is selected from

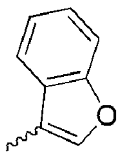, or 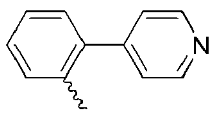,

and hydrogen atom(s) of the aryl group therein can be further substituted by a fluorine atom, a chlorine atom, an alkyl group, or an alkoxy group, and the alkoxy group can be further substituted by either group selected from a heterocyclic group and an alkoxy group.

More preferably, in the compound represented by the general formula (I) of the present invention:

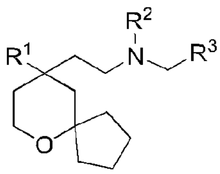

Formula I

$R^1$ is selected from

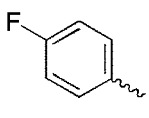, or 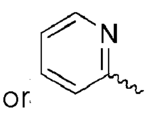;

$R^2$ is selected from a hydrogen atom;

$R^3$ is selected from

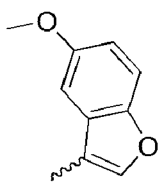, 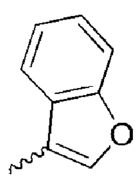, 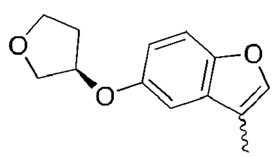, or 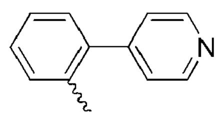.

Even more preferably, in the compound represented by the general formula (I) of the present invention:

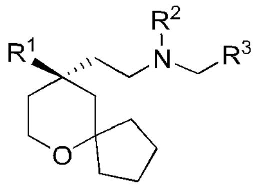

Formula Ia

$R^1$ is selected from

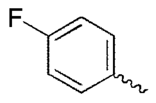, or 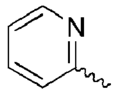;

$R^2$ is selected from a hydrogen atom;

$R^3$ is selected from

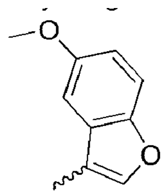, 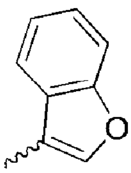, 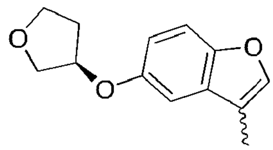, or 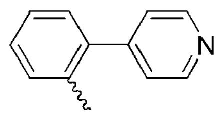.

Among the 6-oxaspiro[4.5]decane derivatives represented by the general formula (I) of the present invention, preferred compounds include, but are not limited to, the following compounds:

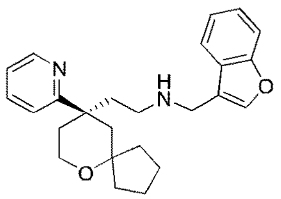, 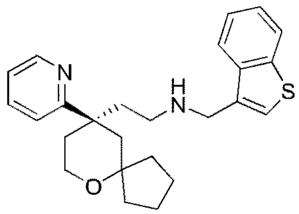, 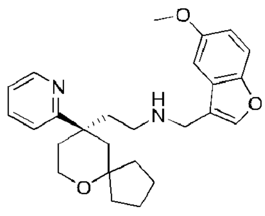, 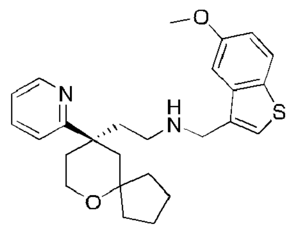,

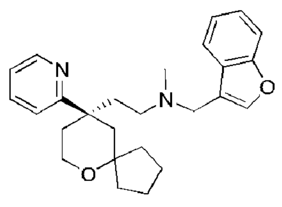, 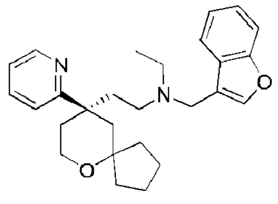, 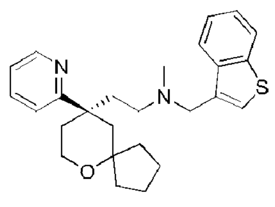, 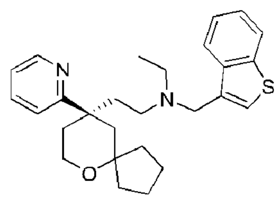,

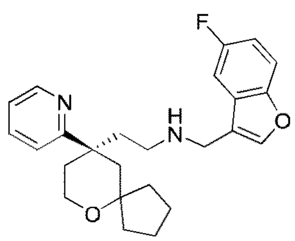, 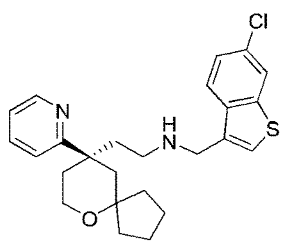, 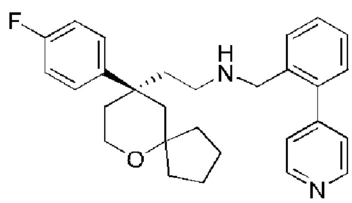,

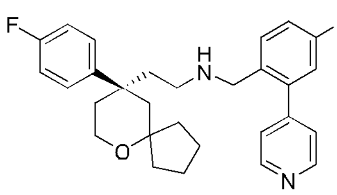, 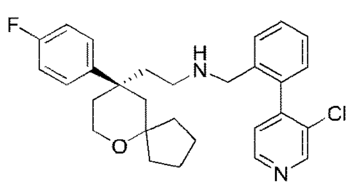, 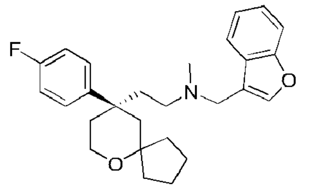,

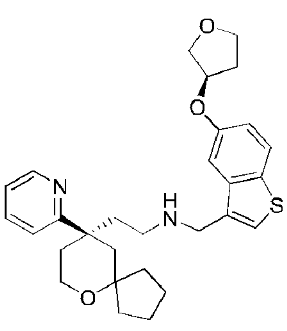, 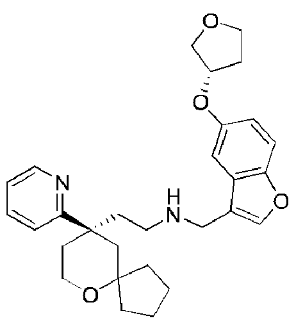, or 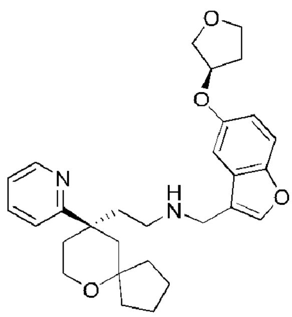.

Further, among the 6-oxaspiro[4.5]decane derivatives represented by the general formula (I) of the present invention, preferred compounds include, but are not limited to, the following compounds:

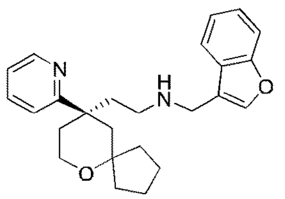, 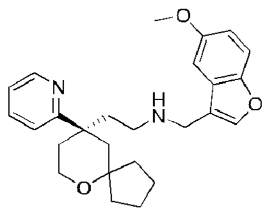, 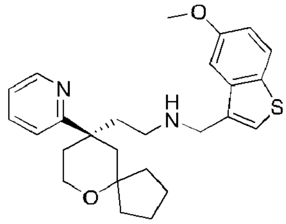,

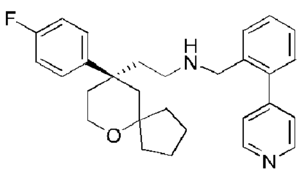, 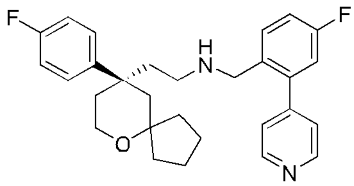, 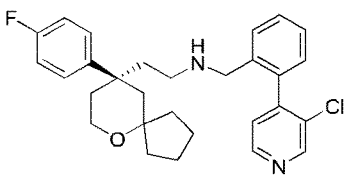,

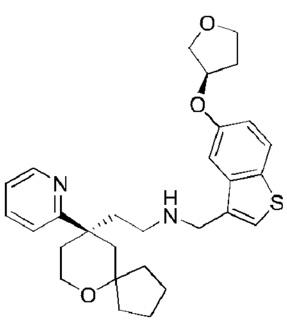, 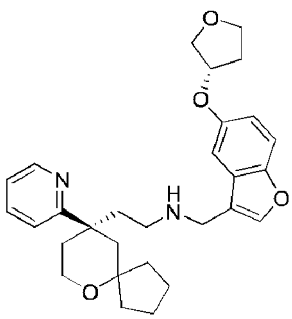, or 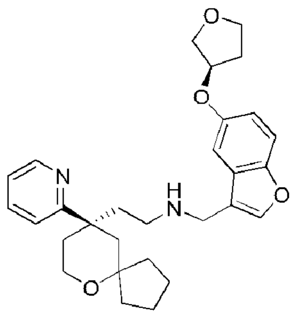.

Another object of the present invention is to provide a preparation method for preparing the above compound of formula (I), comprising the following steps:

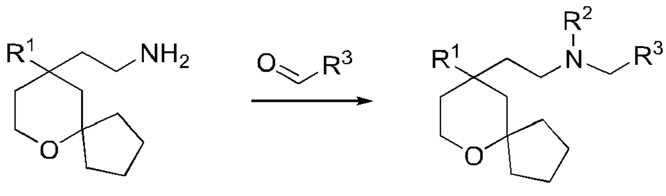

a I

the starting material a reacts with the starting material aldehyde to obtain the target compound I with $R^2$=H; if $R^2$ is an alkyl group, the target compound I can be obtained after further alkylation reaction.

Wherein, in the raw material, $R^1$ is selected from a phenyl group or a pyridyl group, wherein hydrogen atom(s) of the phenyl group or the pyridyl group can be further substituted by a fluorine atom or a chlorine atom; $R^2$ is selected from a hydrogen atom or a $C_1$-$C_3$ alkyl group; $R^3$ is selected from

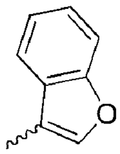 , 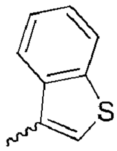 , or

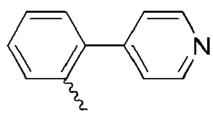 ,

and hydrogen atom(s) of the aryl group therein can be further substituted by a fluorine atom, a chlorine atom, an alkyl group, a heterocyclic group, or an alkoxy group, and the alkoxy group can be further substituted by either group selected from a heterocyclic group and an alkoxy group.

Further, $R^1$ is selected from a phenyl group or a pyridyl group, wherein hydrogen atom(s) of the phenyl group or the pyridyl group can be further substituted by a fluorine atom or a chlorine atom; $R^2$ is selected from a hydrogen atom, a methyl group or an ethyl group; $R^3$ is selected from

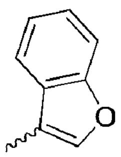 or 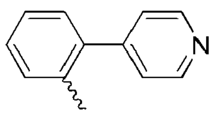 ,

and hydrogen atom(s) of the aryl group therein can be further substituted by a fluorine atom, a chlorine atom, an alkyl group, or an alkoxy group, and the alkoxy group can be further substituted by either group selected from a heterocyclic group and an alkoxy group. Even further, $R^1$ is selected from

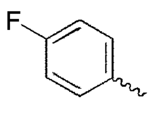 or 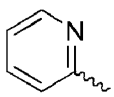 ;

$R^2$ is selected from a hydrogen atom; $R^3$ is selected from

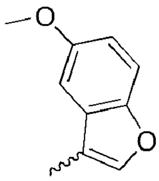, 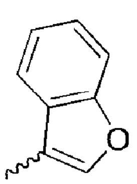, 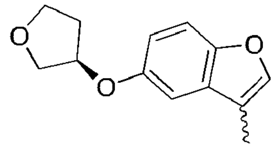,

or

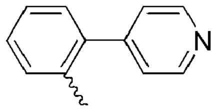.

Preferably, the present invention provides the preparation method for preparing the compound of the above formula (Ia), comprising the following steps:

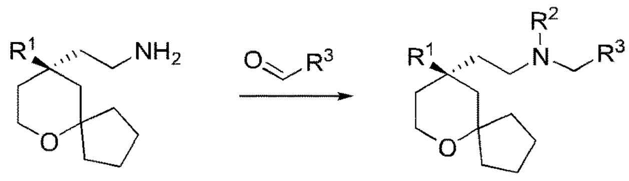

a'　　　　Ia

wherein $R^1$ is selected from

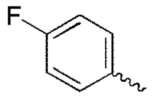 or 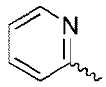;

$R^2$ is selected from a hydrogen atom; $R^3$ is selected from

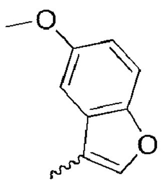, 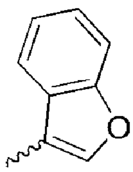, 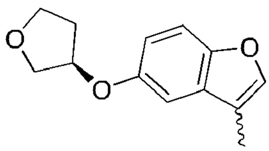, or 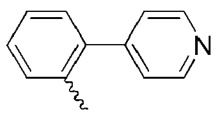;

the starting material a' reacts with the material aldehyde to obtain the target compound Ia.

Another object of the present invention is to provide use of the compound represented by the above general formula (I) in the preparation of μ receptor agonist drugs, specifically, the use in the preparation of analgesic drugs, and more specifically, to provide use of the compound represented by the above general formula (I) in the preparation of a drug suitable for moderate to severe pain. Through the *in vitro* effect test on δ, κ and μ receptors, the compound of the present invention shows excellent μ receptor selectivity and agonistic activity; through the efficacy test regarding the influence on the rat thermal radiation tail-flick model, the compound of the present invention can still show good analgesic effect 3h after administration; through the research test on the pharmacokinetics of normal rats, the compound of the present invention shows excellent pharmacokinetic characteristics; and through the test of the influence on the expulsion time of glass beads from mice colon, the compound of the present invention has a lower prolonging effect on glass bead expulsion from mice than the positive compounds after a single tail vein administration, demonstrating less inhibitory effect on colon function in mice, indicating lower clinical side effects of constipation.

## EMBODIMENTS OF THE INVENTION

The present invention is further described below with reference to the examples, which are, however, not intended to limit the scope of the present application. Any equivalent substitutions of the present art made according to disclosure of the present application shall fall within the scope of the present invention.

The structures of the compounds are determined by mass spectrometry (MS) or nuclear magnetic resonance ($^1$H NMR).

Nuclear magnetic resonance ($^1$H NMR) shifts (δ) are given in parts per million (ppm); the measurements of the nuclear magnetic resonance ($^1$H NMR) are performed with a Bruker AVANCE-400 nuclear magnetic resonance spectrometer, with deuterated chloroform ($CDCl_3$) as the measurement solvent and tetramethylsilane (TMS) as the internal standard, and chemical shifts are given in $10^{-6}$ (ppm).

Mass spectrometry (MS) measurements are performed with a FINNIGAN LCQAd (ESI) mass spectrometer (manufacturer: Therm, model: Finnigan LCQ advantage MAX).

Thin-layer silica gel chromatography uses Yantai Huanghai HSGF254 or Qingdao GF254 silica gel plate.

Column chromatography generally uses Yantai Huanghai silica gel 200-300 mesh silica gel as the carrier.

Unless particularly specified in the present invention, the reactions mentioned in the present invention are all carried out under nitrogen atmosphere.

The term "nitrogen atmosphere" in the present invention refers to, for example, connecting the reaction flask to a nitrogen balloon of 1 L volume.

Unless particularly specified in the present invention, the solution mentioned in the reaction of the present invention is water solution.

The term "room temperature" in the present invention refers to a temperature between 10°C and 25°C.

In one embodiment, the present invention relates to a 6-oxaspiro[4.5]decane derivative having the structure represented by the following general formula (I):

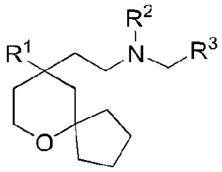

Formula I

or its tautomer, mesomer, racemate, enantiomer, diastereomer or mixture form thereof;
wherein,

$R^1$ is selected from a phenyl group or a pyridyl group, wherein hydrogen atom(s) of the phenyl group or the pyridyl group can be further substituted by fluorine atom or chlorine atom;
$R^2$ is selected from a hydrogen atom or a $C_1$-$C_3$ alkyl group;
$R^3$ is selected from

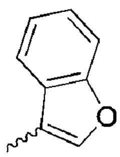
,
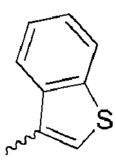
, or
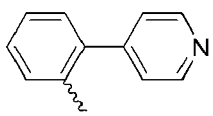
,

and hydrogen atom(s) of the aryl group therein can be further substituted by a fluorine atom, a chlorine atom, an alkyl group, a heterocyclic group, or an alkoxy group, and the alkoxy group can be further substituted by either group selected from a heterocyclic group and an alkoxy group.

In a preferred embodiment, $R^1$ is selected from a phenyl group or a pyridyl group, wherein hydrogen atom(s) of the phenyl group or the pyridyl group can be further substituted by a fluorine atom or a chlorine atom; $R^2$ is selected from a hydrogen atom, a methyl group or an ethyl group; $R^3$ is selected from

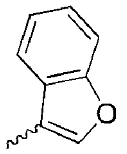
,

or

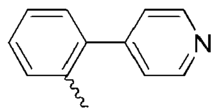
,

and hydrogen atom(s) of the aryl group therein can be further substituted by a fluorine atom, a chlorine atom, an alkyl group, or an alkoxy group, and the alkoxy group can be further substituted by either group selected from a heterocyclic group and an alkoxy group.

In a more preferred embodiment, $R^1$ is selected from

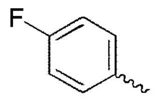 or 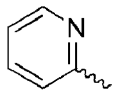 ; $R^2$ is

selected from hydrogen atom; $R^3$ is selected from

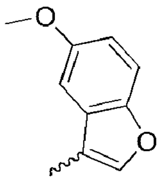 , 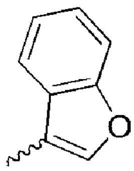 ,

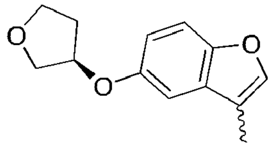 , or 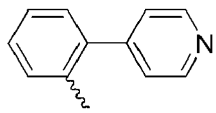 .

EXAMPLES

Example 1 Preparation of Compound 1

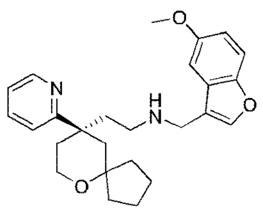

The preparation scheme is shown below:

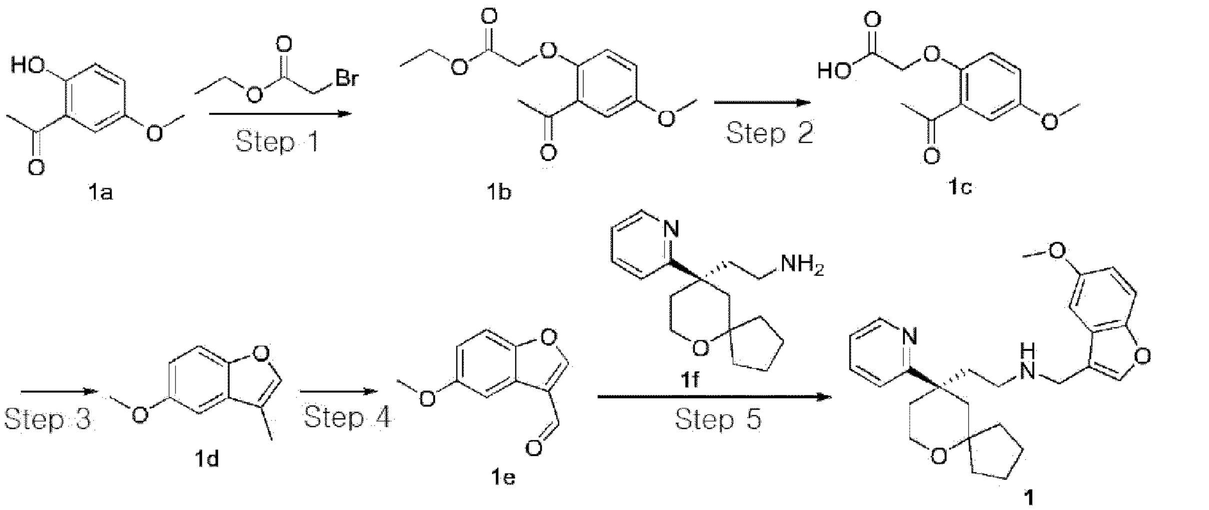


1a 1b 1c 1d 1e 1f 1

Step 1: Preparation of compound 1b

2'-Hydroxy-5'-methoxyacetophenone 1a (30g, 0.18mol) was dissolved in N,N-dimethylformamide (30mL), potassium carbonate (99.8g, 0.72mol) was added thereto, and ethyl bromoacetate (36 g, 0.22 mol) was added under an ice-water bath. The reaction was carried out at room temperature after the addition was completed. After the reaction was completed, water was added to the system to quench the reaction, and extraction was performed 3 times with ethyl acetate. The organic phases were combined, then washed once with water and saturated brine, respectively, dried over anhydrous sodium sulfate, and then filtered by suction. The filtrate was concentrated to obtain compound 1b (45.4 g, off-white solid) with a yield of 99.6%.
MS m/z (ES): 253.1 [M+1]

Step 2: Preparation of compound 1c

Compound 1b (45.4 g, 0.18 mol) was dissolved in methanol (315 mL), and a solution of sodium hydroxide (28.9 g, 0.72 mol) and water (135 mL) was added under an ice-water bath. The reaction was carried out at room temperature after the addition was completed. After the reaction was completed, the solvent in the reaction system was removed through concentration, water was added thereto for dissolving, and pH value thereof was adjusted to 4-5 with diluted hydrochloric acid, and then extraction was performed three times with ethyl acetate. The organic phases were combined, then washed once with water and saturated brine, respectively, dried over anhydrous sodium sulfate, and then filtered by suction. The filtrate was concentrated to obtain compound 1c (39.1 g, off-white solid) with a yield of 96.8%.
MS m/z (ES): 225.1 [M+1]

Step 3: Preparation of compound 1d

Compound 1c (39.1 g, 0.17 mol) was dissolved in acetic anhydride (780 mL), and sodium acetate (200 g, 2.4 mol) was added thereto at room temperature. The reaction was carried out at 110°C after the addition was completed. After the reaction was completed, ice-water was added to the system under an ice-water bath to quench the reaction, and the reaction was stirred for 30min, then the pH value thereof was adjusted to 8-9 with sodium hydroxide, and extraction was performed 4 times with petroleum ether. The organic phases were combined, then washed once with water and saturated brine, respectively, dried over anhydrous sodium sulfate, and then filtered by suction. The filtrate was concentrated to obtain compound 1d (24 g, yellow oily substance) with a yield of 84.8%.

Step 4: Preparation of compound 1e

Compound 1d (4.0 g, 24.7 mmol) was dissolved in 1,4-dioxane (40 mL), and selenium dioxide (9.8 g, 88 mmol) was added thereto at room temperature. The reaction was carried out at 110 °C after the addition was completed. After the reaction was completed, water was added to the system to quench the reaction, and extraction was performed 3 times with ethyl acetate. The organic phases were combined, then washed once with water and saturated brine, respectively, dried over anhydrous sodium sulfate, and then filtered by suction. The filtrate was concentrated to obtain an oily substance, which was purified by column chromatography (ethyl acetate: petroleum ether=1: 15) to obtain compound

1e (2.4 g, orange-red oily substance) with a yield of 55.2%.
MS m/z (ES): 177.1 [M+1]

Step 5: Preparation of compound 1

(R)-2-(9-(pyrid-2-yl)-6-oxaspiro[4.5]decan-9-yl)ethylamine 1f (3 g, 11.5 mmol) was dissolved in dichloromethane (20 mL), and compound 1e (2.1 g, 11.9 mmol), and anhydrous sodium sulfate (6.4 g, 46 mmol) were added thereto. The reaction was carried out at room temperature after the addition was completed. After 12 h of reaction, dichloromethane in the reaction system was removed through concentration, then methanol (20 mL) was added for dissolving and sodium borohydride (0.44 g, 11.6 mmol) was added in portions under an ice-water bath. After the reaction was completed, water was added to the system to quench the reaction, and then methanol in the reaction system was removed through concentration to obtain an oily substance. The oily substance was dissolved with water and ethyl acetate, and extracted three times with ethyl acetate. The organic phases were combined, then washed once with water and saturated brine, respectively, dried over anhydrous sodium sulfate, and then filtered by suction. The filtrate was concentrated to obtain a yellow oily substance, which was purified by column chromatography (dichloromethane: methanol=20: 1) to obtain compound 1 (1.05 g, yellow oily substance) with a yield of 21.7% and a purity of 98.28%.
MS m/z (ES): 421.2[M+1]

1H NMR (400 MHz, CDC13) δ 8.53 - 8.52 (m, 1H), 7.61 - 7.56 (m, 1H), 7.40 (s, 1H), 7.32 (d, J = 8.9 Hz, 1H), 7.27 - 7.25 (m, 1H), 7.10 - 7.07 (m, 1H), 6.99 (d, J = 2.5 Hz, 1H), 6.89 - 6.86 (m, 1H), 3.84 (s, 3H), 3.76 - 3.73 (m, 4H), 2.61 - 2.54 (m, 1H), 2.45 - 2.41 (m, 1H), 2.36 - 2.33 (m, 1H), 2.22 - 2.15 (m, 1H), 2.05 - 1.98 (m, 1H), 1.91 (d, J = 13.7 Hz, 1H), 1.82 - 1.62 (m, 4H), 1.54 - 1.37 (m, 4H), 1.13 - 1.08 (m, 1H), 0.72 - 0.64 (m, 1H).

Example 2 Preparation of compound 2

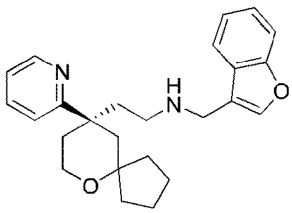

The preparation scheme is shown below:

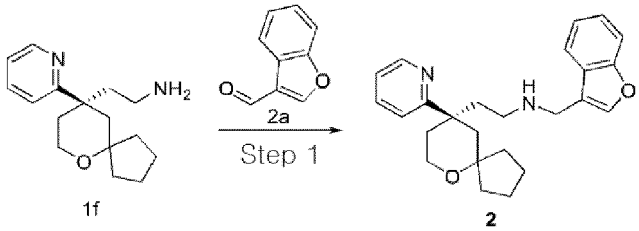


1f

2

Step 1: Preparation of compound 2

(R)-2-(9-(pyrid-2-yl)-6-oxaspiro[4.5]decan-9-yl)ethylamine 1f (999 mg, 3.6 mmol) was dissolved with dichloromethane (10 mL), and benzofuran-3-carbaldehyde 2a (0.53 g, 3.6 mmol) and anhydrous sodium sulfate (2.1 g, 14.4 mmol) were added thereto. The reaction was carried out at room temperature after the addition was completed. After 12 h of reaction, dichloromethane in the reaction system was removed through concentration, then methanol (7 mL) was added for dissolving and sodium borohydride (273 mg, 7.2 mmol) was added in portions under an ice-water bath. After the reaction was completed, methanol in the reaction system was removed through concentration, then water and ethyl acetate was added thereto for dissolving, and the solution were separated. The aqueous phase was extracted twice with ethyl acetate. The organic phases were combined, then washed once with water and saturated brine, respectively, dried over anhydrous sodium sulfate, and then filtered by suction. The filtrate was concentrated to obtain a yellow oily substance, which was purified by column chromatography (dichloromethane: methanol=25: 1) to obtain compound 2 (0.63 g, yellow oily substance) with a yield of 44.8% and a purity of 96.95%.

MS m/z (ES): 391.2[M+1]

$^1$H NMR (400MHz, CDCl3) δ 8.56-8.54 (m, 1H), 7.61-7.57 (m, 1H), 7.51-7.43 (m, 2H), 7.39 (s, 1H), 7.29-7.25 (m, 2H), 7.23-7.19 (m, 1H), 7.11-7.08 (m, 1H), 3.77-3.74 (m, 2H), 3.73 (s, 2H), 2.58-2.51 (m, 1H), 2.46-2.42 (m, 1H), 2.37-2.34 (m, 1H), 2.19-2.12 (m, 1H), 2.02-1.96 (m, 1H), 1.92 (d, J=13.7Hz, 1H), 1.76-1.67 (m, 4H), 1.55-1.40 (m, 4H), 1.14-1.09 (m, 1H), 0.73-0.65 (m, 1H).

Example 3 Preparation of compound 3

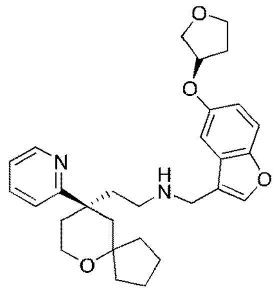

The preparation scheme is shown below:

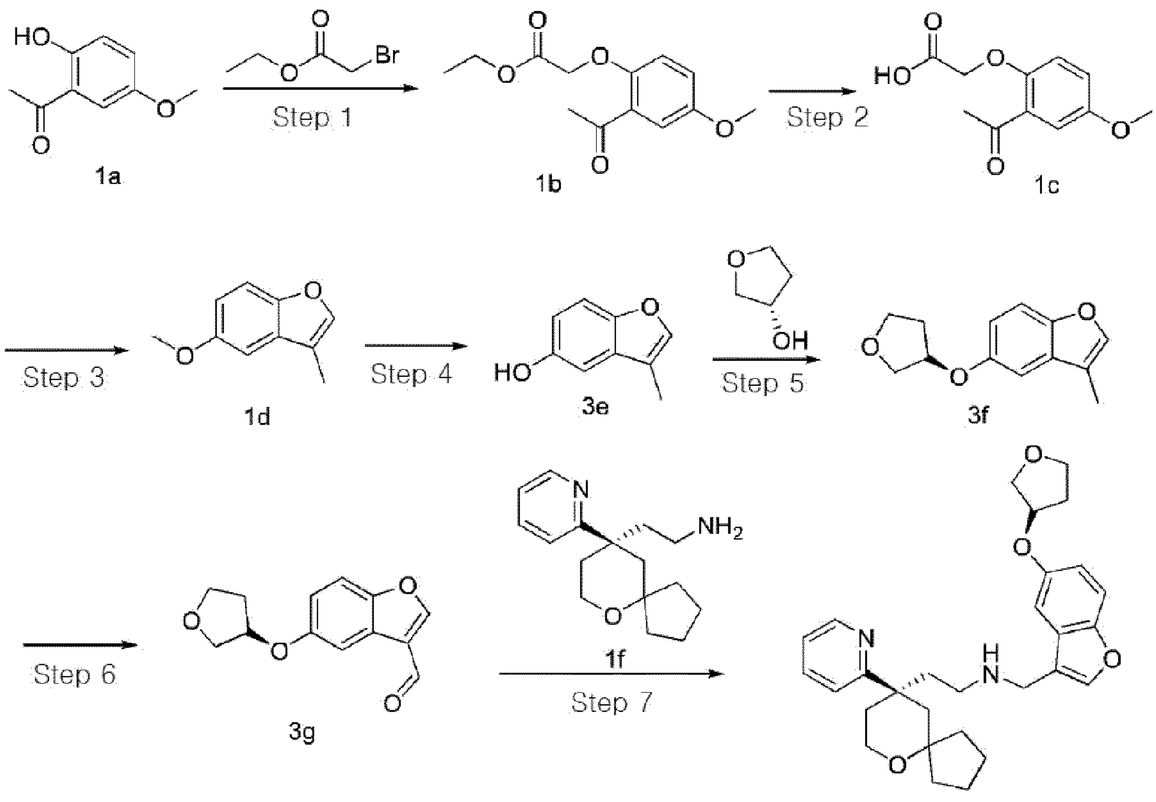


3

Step 1 is the same as step 1 in Example 1;

Step 2 is the same as step 2 in Example 1;

Step 3 is the same as step 3 in Example 1;

Step 4: Preparation of compound 3e

Compound 1d (2.1 g, 12.9 mol) was dissolved with dichloromethane (21 mL), and boron tribromide (6.5 g, 25.9 mol) was added dropwise thereto at 0 to 5 °C. The reaction was carried out at 0 to 5 °C after the addition was completed. The reaction was monitored by TLC. After the reaction of the raw materials was completed, water was added to the reaction system to quench the reaction, and suction filtration (with diatomaceous earth being filled) was performed. The filter cake was washed with dichloromethane, and the filtrate was separated. The aqueous phase was then extracted twice with dichloromethane. The organic phases were combined, then washed once with water, dried over anhydrous sodium sulfate, and then filtered by suction. The filtrate was concentrated to obtain 1.6 g of compound 3e as a yellow oily substance with a yield of 83.4%.

ESI-MS: m/z=149.1[M+1]

Step 5: Preparation of compound 3f

Compound 3e (1.6 g, 10.8 mol) and S-3-hydroxytetrahydrofuran (0.95 g, 10.8 mol) were dissolved with dichloromethane (16 mL), and triphenylphosphine (3.1 g, 11.9 mol) and DIAD (2.4 g, 11.9 mol) were added thereto at 0 to 5 °C. The reaction was carried out at room temperature after the addition was completed. The reaction was monitored by TLC. After the reaction of the raw materials was completed, water was added to the reaction system to quench the reaction, and extraction was performed twice with ethyl acetate. The organic phases were combined, then washed once with water and saturated brine, respectively, dried over anhydrous sodium sulfate, and then filtered by suction. The filtrate was concentrated to obtain a yellow oily substance, which was purified by column chromatography (ethyl acetate: petroleum ether=1: 5) to obtain 1.8 g of compound 3f as a yellow oily substance with a yield of 76.4%.

ESI-MS: m/z=219.1[M+1]

Step 6: Preparation of compound 3g

Compound 3f (1.8 g, 8.2 mmol) was dissolved in 1,4-dioxane (18 mL), and selenium dioxide (4.6 g, 41.2 mmol) was added thereto at room temperature. The reaction was carried out at 110 °C after the addition was completed. The reaction was monitored by TLC. After the reaction of the raw materials was completed, water was added to the system to quench the reaction, and extraction was performed 3 times with ethyl acetate. The organic phases were combined, then washed once with water and saturated brine, respectively, dried over anhydrous sodium sulfate, and then filtered by suction. The filtrate was concentrated to obtain a oily substance, which was purified by column chromatography (ethyl acetate: petroleum ether=1: 5) to obtain 0.85g of compound 3g as an orange-red oily substance with a yield of 44.3%.

ESI-MS: m/z=233.1[M+1]

Step 7: Preparation of compound 3

Compound 3g (0.85g, 3.7mmol) was dissolved with dichloromethane (10mL), and (R)-2-(9-(pyrid-2-yl)-6-oxaspiro[4.5]decan-9-yl)ethylamine 1f (0.95 g, 3.7 mmol) and anhydrous sodium sulfate (2.1 g, 14.6 mmol) were added thereto. The reaction was carried out at room temperature after the addition was completed. After 12 hours of reaction, anhydrous sodium sulfate was added and stirred for 30 minutes, and suction filtration was performed. The filtrate was concentrated to obtain an oily substance, which was then dissolved with methanol (20mL), and sodium borohydride (0.14 g, 3.7 mmol) was added in portions at -10°C to -5°C. The reaction was monitored by LC-MS. After the reaction of the raw materials was completed, water was added to the reaction system to quench the reaction, and then methanol in the reaction system was removed through concentration to obtain an oily substance. The oily substance was dissolved with water and ethyl acetate, and was extracted three times with ethyl acetate. The organic phases were combined, then washed once with water and saturated brine, respectively, dried over anhydrous sodium sulfate, and then filtered by suction. The filtrate was concentrated to obtain a yellow oily substance, which was purified by column chromatography (dichloromethane: methanol=20: 1) to obtain 0.53 g of compound 3 as a yellow oily substance, with a yield of 30.2% and a purity of 98.88%.

ESI-MS: m/z=477.3[M+1]

$^1$HNMR (400 MHz, CDC13) $\delta$ 8.55 - 8.53 (m, 1H), 7.62 - 7.58 (m, 1H), 7.38 (s, 1H), 7.34 - 7.28 (m, 2H), 7.11 - 7.08 (m, 1H), 6.96 (d, J = 2.5 Hz, 1H), 6.87 - 6.84 (m, 1H), 4.96 - 4.93 (m, 1H), 4.04 - 3.98 (m, 3H), 3.94 - 3.89 (m, 1H), 3.76 - 3.74 (m, 2H), 3.69 (s, 2H), 2.59 - 2.52 (m, 1H), 2.46-2.42 (m, 1H), 2.37-2.33 (m, 1H), 2.20-2.12 (m, 3H), 2.01

- 1.96 (m, 1H), 1.92 (d, J = 13.7 Hz, 1H), 1.79 - 1.64 (m, 4H), 1.54 - 1.38 (m, 4H), 1.14 - 1.09 (m, 1H), 0.72 - 0.65 (m, 1H).

Example 4 Preparation of compound 4

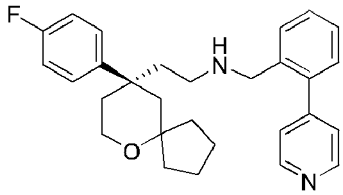

The preparation scheme is shown below:

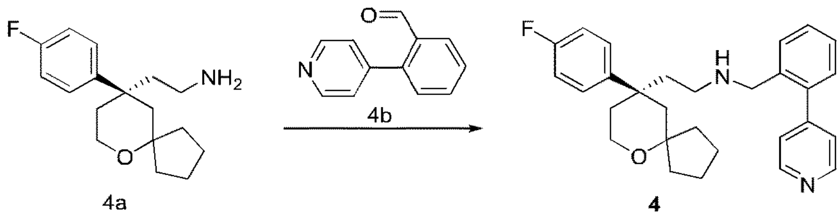


4a

**4**

(R)-2-(9-(4-fluorophenyl)-6-oxaspiro[4.5]decan-9-yl)ethylamine 4a (666 mg, 2.4 mmol) was dissolved with dichloromethane (7 mL), and 2-(4-pyridyl)benzaldehyde 4b (0.44g, 2.4mmol) and anhydrous sodium sulfate (1.4g, 9.6mmol) were added thereto. The reaction was carried out at room temperature after the addition was completed. After 12 h of reaction, dichloromethane in the reaction system was removed through concentration, then methanol (7 mL) was added for dissolving, and sodium borohydride (182 mg, 4.8 mmol) was added in portions under an ice-water bath. After the reaction was completed, methanol in the reaction system was removed through concentration, and then water and ethyl acetate were added for dissolving, and the solution was separated. The aqueous phase was extracted twice with ethyl acetate. The organic phases were combined, then washed once with water and saturated brine, respectively, dried over anhydrous sodium sulfate, and then filtered by suction. The filtrate was concentrated to obtain a yellow oily substance, which was purified by column chromatography (dichloromethane: methanol=25: 1) to obtain compound 4 (0.46 g, yellow oily substance) with a yield of 42.7% and a purity of 99.59%.
MS m/z (ES): 445.3[M+1]

$^1$H NMR (400 MHz, $CDCl_3$) $\delta$ 8.62 - 8.60 (m, 2H), 7.35 - 7.30 (m, 3H), 7.27 - 7.25 (m, 2H), 7.21 - 7.17 (m, 3H), 7.01 - 6.97 (m, 2H), 3.74 - 3.68 (m, 2H), 3.51 (s, 2H), 2.32 - 2.25 (m, 1H), 2.17 - 2.13 (m, 1H), 2.03 - 1.96 (m, 2H), 1.87 (d, *J* = 13.9 Hz, 1H), 1.78 - 1.57 (m, 6H), 1.50 - 1.40 (m, 4H), 0.85 - 0.77 (m, 1H).

Pharmacological and toxicological evaluation

Test drug:

Positive control drug: TRV130, provided by Chengdu Easton Biopharmaceuticals Co., Ltd., orange-yellow oily substance, purity: 99.95%;
The compound of Example 1: prepared in Example 1, provided by the Synthesis Laboratory of Chengdu Easton Biopharmaceuticals Co., Ltd., yellow oily substance, purity: 98.28%;
The compound of Example 2: prepared in Example 2, provided by the Synthesis Laboratory of Chengdu Easton Biopharmaceuticals Co., Ltd., yellow oily substance, purity: 96.95%;
The compound of Example 3: prepared in Example 3, provided by the Synthesis Laboratory of Chengdu Easton Biopharmaceuticals Co., Ltd., yellow oily substance, purity: 98.88%;
Example 4 Compound: prepared in Example 4, provided by the Synthesis Laboratory of Chengdu Easton Biopharmaceuticals Co., Ltd., yellow oily substance, purity: 99.59%.

**Experimental Example 1** *In vitro* test of the activity of each compound of formula I on different receptors

1. Test purpose: to detect the agonistic activity of compound samples utilizing MOR (Mu opioid Receptor), KOR (Kappa opioid receptor), and DOR (Delta Opioid Receptor) stably transfected cell lines and using the established cAMP assay detection platform.

2. Test method

2.1 Compound formulation

a. All samples were dissolved with DMSO to a storage concentration of 30 mM.

b. A sample dilution sequence was prepared on a 384-well LDV plate. The first concentration point is 6 mM, with a 3.162-fold serial dilution and a total of 11 concentration points.

c. The sample dilution sequence was transferred to an test plate (Corning-3824) using the Echo machine, corresponding to transfer of 50 nL per well.

d. 50 nL of HPE or ZPE was transferred to the corresponding location of the test plate using the Echo machine.

2.2 Test operation of cAMP agonistic activity detection

a. Assay buffer and stimulation buffer (STB) required for the test were formulated.
b. 5μL of STB was firstly added to each well of the test plate (with 50nL of compound already in the well), and then 5μL of cell suspension was added thereto.
c. The test plate was incubated in a constant temperature incubator at 37°C.
d. A standard concentration curve of cAMP was created. The first concentration point was 2848nM, with a 4-fold serial dilution and a total of 16 detection concentrations, and each concentration point was in triplicate.
e. cAMP detection reagent (Cisbio #62AM4PEJ) was added to the sample test plate and cAMP standard concentration test plate. 5μL of D2 reagent was added first, and then 5μL of Cryptate reagent was added.
f. The test plates were read on the Envision after being left for 60 minutes at room temperature.

3. Test results

Table 1 The agonistic effect of each compound in the Experimental Example on different opioid receptors

| Compounds in the Experimental Example | $EC_{50}$ (uM) | | |
|---|---|---|---|
| | μ | K | δ |
| TRV130 | 0.0051 | 5.77 | 3.14 |
| Example 1 | 0.00021 | 51.4 | 65.3 |
| Example 2 | 0.0029 | 39.8 | 22.2 |
| Example 3 | 0.00056 | 56.8 | 32.5 |
| Example 4 | 0.00045 | 54.5 | 42.7 |

4. Conclusion

All the compounds in the Experimental Example of the present invention had a good agonistic effect on μ opioid receptor, while the agonistic effects on κ and δ opioid receptors were obviously weaker.

Compared with the positive compound (TRV130), the $EC_{50}$ values of Example 1, Example 3, and Example 4 against μ receptor were about 1/10 of that of TRV130, and the $EC_{50}$ values against κ and δ receptors are about 10 times or higher of that of TRV130, indicating that the agonistic effect of Example 1, Example 3 and Example 4 was obviously superior to that of the positive drug, and the selectivity was better.

The $EC_{50}$ value of Example 2 was the same order of magnitude as that of TRV130, and the $EC_{50}$ values on κ and δ receptors were about 7 times that of TRV130, indicating that the agonistic effect of Example 2 was equivalent to that of positive drug, but the selectivity was better.

In summary, the compounds in the Experimental Example of the present invention had relatively good agonistic effect on the μ-opioid receptor, and the selectivity was better.

**Experimental Example 2** Test for the effect of the compound of formula I on the thermal radiation tail-flick pain threshold time in rats

1. Test purpose: to measure the effect of each compound sample on the pain threshold of rats by using the rat thermal radiation tail flick method.

2. Test method

(1) Dosage and route of administration

Administered via tail vein injection at an equal dose of 0.7 mg/kg;

(2) Operation method

The rats (SD rats, SPF grade, with body weight of 220-250g, purchased from Beijing Vital River Laboratory Animal Technology Co., Ltd.) were firstly fixed in a special plastic cylinder, so that their tails were exposed and drooped naturally. Test was performed after the animals were quiet. An 8.75mm projection bulb (34W, adjustable) could be used as the radiant heat source, which was focused by a lens and emitted a beam with a diameter of about 4mm, irradiating the skin equivalent to the junction of the middle and lower thirds of the tail (the light source and the skin of the tail must be closely attached); an electronic timer connected in parallel with the light source was used to record the duration of irradiation synchronously, that is, when the irradiation began, the timing was automatically started at the same time, and when the animal's tail showed obvious escape response, the light source was automatically turned off and the timing was stopped at the same time. The measured time interval was the tail-flick response latency, which is 3 s to 5 s in normal rats, and those with greater than 5 s or less than 3 s were excluded. Then, measurement was made every 5min, and the mean of three measurements was taken as the basic nociceptive threshold. If the animal stayed more than 10 s under analgesic effect, the irradiation was stopped and 10 s was used as the upper limit of the tail flick latency to avoid burning the skin by the toolong irradiation. After the rats were given a certain dose of the drug, the pain thresholds of the rats were measured at different times.

(3) Detection indicators and statistical methods

After the rats were given a certain dose of the drug, the pain threshold of the rats was measured at 0.5 h, 1 h, 2 h, and 3 h. The maximum percentage of analgesia (% MPE) after administration was calculated, of which the calculation formula was as follows: MPE(%)=(pain threshold after administration - pain threshold before administration) / (10s - pain threshold before administration))×100%

3. Test results

Table 2 Maximum percentage of analgesia (MPE (%), Mean ± SEM, n=10) at different times after administration of each compound

| Groups | 0.5 h | 1 h | 2 h | 3h |
|---|---|---|---|---|
| TRV130 | 100.00±0. 00 | 88.69±1.5 2 | 70.01±8.01 | 49.32±7.12 |
| Example 1 | 1 00.00±0. 00 | 89.95±7.0 6 | 86.51±10.06※※ | 65.91±.5.76※※ |
| Example 2 | 100.00±0.00 | 82.81±9.77 | 79.23±10.51※ | 55.50±6.85※※ |
| Example 3 | 100.00±0. 00 | 86.89±7.34 | 82.95±8.69※※ | 61.46±8.58※※ |
| Example 4 | 100.00±0. 00 | 85.79±9.2 5 | 83.78±11.31※※ | 60.92±2.45※※ |
| Note: Compared with TRV130, ※※ P<0.01, ※ P<0.05 | | | | |

Each compound in this Experimental Example had significantly prolonged effect on the thermal radiation tail-flick pain threshold time in rats.

0.5h after administration, the MPE of each compound in this Experimental Example reached 100%, which was consistent with the positive compound (TRV130);

1 h after administration, the MPEs of compounds in this Experimental Example, i.e., compounds of Example 1, Example 2, Example 3, and Example 4, were 89.95±7.06, 82.81 ±9.77, 86.89±7.34, 85.79±9.25, respectively, which were substantially equivalent to the positive compounds (with MPE of 88.69±1.52), and there was no statistical difference;

2 h after administration, compared with the positive compound (with MPE of 70.01 ±8.01), Example 1, Example 3 and Example 4 had a very obvious effect on prolonging the pain threshold time, and the difference was statistically significant ($P<0.01$), and the corresponding MPEs were 86.51±10.06, 82.95±8.69, 83.78±11.31, respectively; while Example 2 had an obvious effect on prolonging pain threshold time compared with the positive compound, and the difference was statistically significant ($P<0.05$), and the corresponding MPE was 79.23±10.51;

3 h after administration, compared with the positive compound (with MPE of 49.32±7.12), Example 1, Example 3 and Example 4 had a very obvious effect on prolonging the pain threshold time, and the difference was statistically significant ($P<0.01$), and the corresponding MPEs were 65.91 ±5.76, 61.46±8.58, 60.92±2.45, respectively; while Example 2 had an obvious effect on prolonging pain threshold time compared with the positive compound, and the difference was statistically significant ($P<0.05$), and the corresponding MPE was 55.50±6.85;

The above results indicated that, at the same dose, compared with the positive compound, Example 1, Example 2, Example 3, and Example 4 all had analgesic effect with a longer duration, and the prolonged effect thereof had a statistical advantage.

**Experimental Example 3** Pharmacokinetic test of compound of formula I after single intravenous injection

1. Test purpose: to investigate the pharmacokinetic parameters of each compound in the Experimental Example after a single tail vein administration.

2. Test method

(1) Dosage and route of administration

Administered via tail vein at an equal dose of 2 mg/kg;

(2) Blood collection point design

Blood was collected from the jugular vein before administration and 5min, 10min, 15min, 20min, 30min, 45min, 1h, 2h, 4h, 6h, 8h, 24h after administration. 200 μL of whole blood was taken at each time point and was placed in an anticoagulation tube containing EDTA-$K_2$, and centrifuged at 2000g at a low temperature of 4°C to separate the plasma. The plasma was transferred to a microcentrifuge tube, and stored in a -80°C refrigerator for later use.

(3) Sample detection

By establishing a reliable LC/MS/MS analysis method, the blood concentration of each sample was detected, and the pharmacokinetic parameters of each compound were calculated.

3. Test results

Table 3. Main pharmacokinetic parameters of single tail vein injection (n=3)

| Parameters | $T_{1/2}$ (h) | Tmax (h) | $C_{max}$ ($ng \cdot mL^{-1}$) | $AUC_{last}$ ($h \cdot ng \cdot mL^{-1}$) |
|---|---|---|---|---|
| TRV130 | 0.94±0.03 | 0.14±0.10 | 234.67±22.73 | 319.13±30.63 |
| Example 1 | 3.17±0.02※※ | 0.11 ±0.05 | 601.34±32.53※※ | 584.94±25.91※※ |
| Example 2 | 2.29±0.13※※ | 0.15±0.01 | 308.67±43.02※ | 379.46±63.10※ |
| Example 3 | 2.95+0.34※※ | 0.15±0.02 | 543.09±72.77※※ | 532.04±50.57※※ |
| Example 4 | 3.23±0.11※※ | 0.18±0.03 | 551.77±48.69※※ | 563.07±31.76※※ |

Note: Compared with TRV130, ※※ P<0.01, ※ P<0.05;
$T_{1/2}$ of TRV130, Example 1, Example 2, Example 3, Example 4 after administration through single tail vein injection of rats (SD rats, SPF grade, with body weight of 220-250g, purchased from Beijing Vital River Laboratory Animal Technology Co., Ltd.) were 0.94±0.03, 3.17±0.02, 2.29±0.13, 2.95±0.34, 3.23±0.11, respectively; Tmax were 0.14±0.10, 0.11±0.05, 0.15±0.01, 0.15±0.02, 0.18±0.03, respectively; $C_{max}$ were 234.67±22.73, 601.34±32.53, 308.67±43.02, 543.09±72.77, 551.77± 48.69, respectively; $AUC_{last}$ were 319.13±30.63, 584.94±25.91, 379.46±63.10, 532.04±50.57, 563.07±31.76, respectively.

Compared with the positive compound TRV130, $T_{1/2}$ of Example 1, Example 2, Example 3, and Example 4 were longer, with a very significant statistical difference (all with P<0.01), indicating that all the compounds in this Example had longer analgesia effects; meanwhile, $C_{max}$ and $AUC_{last}$ of Example 1, Example 2, Example 3, and Example 4 were higher, wherein Example 1, Example 3, and Example 4 had very significant statistical differences (all with P<0.01), while Example 2 had a significant statistical difference (P<0.05), indicating that the compounds in the Example had better analgesic effects and lower effective doses.

In summary, compared with the positive compounds, the compounds in the Example had the advantages of better efficacy and longer duration of analgesia in the rat thermal radiation tail flick model, wherein Example 1, Example 3, and Example 4 were relatively better.

**Experimental Example 4** Test on the influence of single intravenous injection of compound of formula I on mouse colonic glass bead expulsion time

1. Test purpose: to test the effect of the test compound on the expulsion time of colonic glass beads from mice, and to preliminarily investigate its inhibitory effect on colonic motility.

2. Test method

(1) Dosage and route of administration

Administered via tail vein injection at an equal dose of 1 mg/kg;

(2) Test process

After 6-7 days of adaptive feeding of C57BL/6 mice, the animals fasted with free access to water for 12-16 hours and then randomly divided into 10 groups, 10 mice per group. Each group employed a single administration via tail vein, and the administration volume was 10 mL/kg. The solvent control group was given a vehicle (normal saline) via tail vein. 20 min after the administration, glass beads preheated to 37°C with a diameter of 2 mm were pushed into the rectum at a distance of 2 cm through the anus. Timing was immediately started and the animals were placed individually in the observation cage to observe the time for the glass beads to be expulsed (the observation time limit was 120 min). If the glass beads had not been expulsed by the end of the observation period, the animals were euthanized and the rectum was dissected to confirm that the glass beads were still in the intestinal tract.

(3) Detection indicators and statistical methods

The related indicators were calculated according to the following calculation formula:

Colonic Expulsion Rate (CER) = number of animals that successfully expulsed glass beads during the observation period / total number of animals in this group × 100%;

Expulsion Time Prolonging Rate (ETPR) = (mean expulsion time of test sample group − mean expulsion time of solvent control group) / mean expulsion time of solvent control group × 100%;

Percent Maximum Possible Effect (% Maximum Possible Effect, Percent MPE) = (mean expulsion time of test sample group − mean expulsion time of solvent control group) / (120 − mean expulsion time of solvent control group) × 100%;

Calculation and related statistical processing were performed on the test data using Microsoft Office Excel 2007 and GraphPad Prism software. Unless otherwise specified, the data were shown as mean ± standard error (Mean ± S.E.M.), and the comparison between groups was performed by one-way ANOVA, and the Dunnett's method was used for pairwise comparison test.

3. Test results

Table 4. The mean expulsion time, expulsion time prolonging rate and maximum possible effect percentage of animals in each group (n=10)

| Groups | Mean Expulsion Time (min) | Expulsion Time Prolonging Rate (ETPR) | Percent Maximum Possible Effect (Percent MPE, %) |
|---|---|---|---|
| Vehicle control group | 4.01±0.40 | / | / |
| TRV130 | 37.57±4.31※※ | 836.91% | 28.93 |
| Example 1 | 17.40±5.84※※♦♦ | 333.92% | 11.55 |
| Example 2 | 25.85±5.88※※♦ | 544.64% | 18.83 |
| Example 3 | 18.99±6.55※※♦♦ | 373.57% | 12.91 |
| Example 4 | 19.13±3.93※※♦♦ | 377.06% | 13.04 |
| Note: Compared with the vehicle control group, ※※ P<0.01;<br>Compared with TRV130, ♦♦ P<0.01, ♦ P<0.05; | | | |

Compared with the vehicle control group, the mean expulsion time of each compound in this Experimental Example was significantly prolonged (all with P<0.01), and the expulsion time prolonging rate was also greatly increased, indicating that each compound had a very obvious prolonging effect on the mouse glass bead expulsion after a single tail vein administration.

Compared with the positive compound TRV130, the mean expulsion times of Example 1, Example 3, and Example 4 were very significantly reduced (all with P<0.01), and the expulsion time prolonging rates were also reduced to a relatively large extent, indicating that Example 1, Example 3, and Example 4 had a very significantly lower prolonging effect on mouse glass bead expulsion after single tail vein administration than that of the positive compound.

Compared with the positive compound TRV130, the mean expulsion time of Example 2 was significantly reduced (P < 0.05), and the expulsion time prolonging rate was also decreased to a certain extent, indicating that Example 2 had a significantly lower prolonging effect on mouse glass bead expulsion after single tail vein administration than that of the positive compound.

In summary, after a single tail vein administration, each compound in this Experimental Example had lower prolonging effect on glass bead expulsion in mice than that of the positive compound, demonstrating less inhibitory effect on colon function of mice, and indicating lower clinical side effects of constipation.

The above results indicate that the compounds in the Examples of the present invention exhibit analgesic effects, and it is obvious for the skilled in the art that various modifications and variation can be made to the compounds, compositions and methods of the present invention without departing from the spirit or scope of the present invention. Accordingly, the present invention encompasses modifications and variations of the present invention as long as they are within the scope of the claims and equivalents thereof.

## Claims

1. A compound represented by the general formula (I):

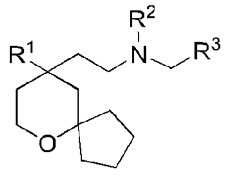

Formula I

wherein,

$R^1$ is selected from a phenyl group or a pyridyl group, wherein hydrogen atom(s) of the phenyl group or the pyridyl group can be further substituted by a fluorine atom or a chlorine atom;
$R^2$ is selected from a hydrogen atom or a $C_1$-$C_3$ alkyl group;
$R^3$ is selected from

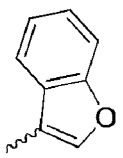 , 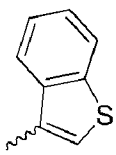 , or 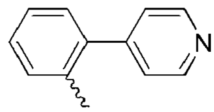 ,

and hydrogen atom(s) of the aryl group therein can be further substituted by a fluorine atom, a chlorine atom, an alkyl group, a heterocyclic group, or an alkoxy group, and the alkoxy group can be further substituted by either group selected from a heterocyclic group and an alkoxy group,
or its tautomer, mesomer, racemate, enantiomer, diastereomer or mixture form thereof.

2. The compound represented by formula (I) according to claim 1, wherein,

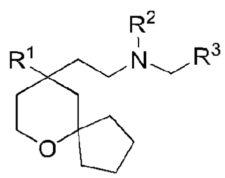

Formula I

$R^1$ is selected from a phenyl group or a pyridyl group, wherein hydrogen atom(s) of the phenyl group or the pyridyl group can be further substituted by a fluorine atom or a chlorine atom;
$R^2$ is selected from a hydrogen atom, a methyl group or an ethyl group;
$R^3$ is selected from

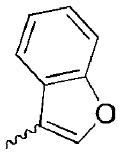, or 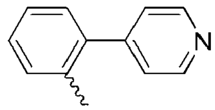,

and hydrogen atom(s) of the aryl group therein can be further substituted by a fluorine atom, a chlorine atom, an alkyl group, or an alkoxy group, and the alkoxy group can be further substituted by either group selected from a heterocyclic group and an alkoxy group.

**3.** The compound represented by formula (I) according to claim 1 or 2, wherein,

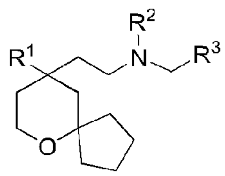

Formula I

$R^1$ is selected from

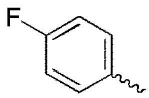, or 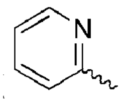;

$R^2$ is selected from a hydrogen atom;
$R^3$ is selected from

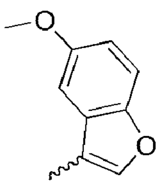, 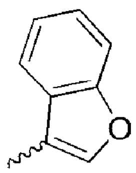, 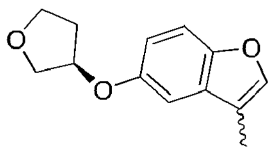, or 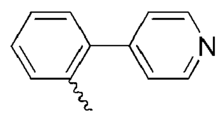.

**4.** The compound represented by formula (I) according to any one of claims 1 to 3, wherein the compound represented by formula (I) has the following structure:

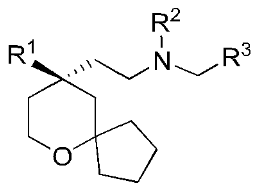

Formula Ia

$R^1$ is selected from

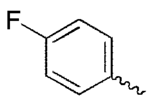, or 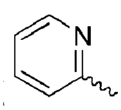;

$R^2$ is selected from a hydrogen atom;
$R^3$ is selected from

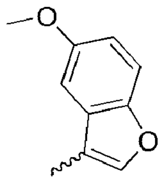, 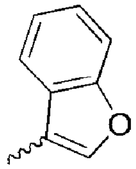, 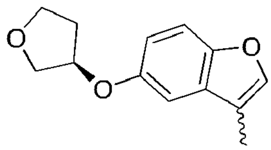, or 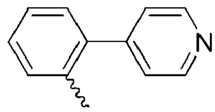.

**5.** The compound represented by formula (I) according to any one of claims 1 to 4, wherein the compound is selected from:

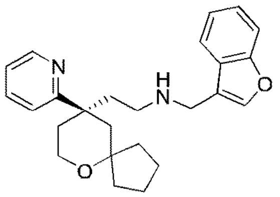, 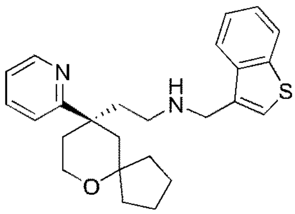, 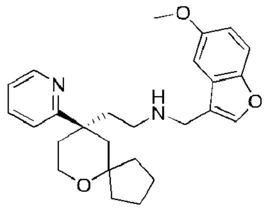, 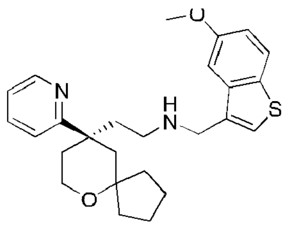,

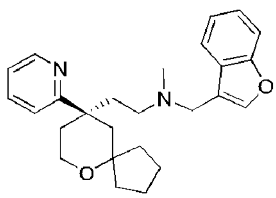, 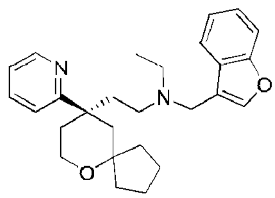, 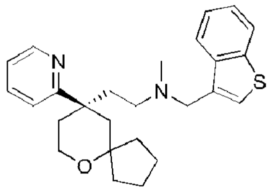, 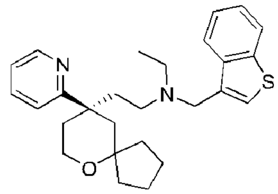,

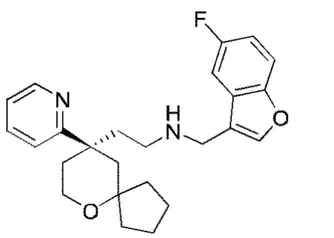, 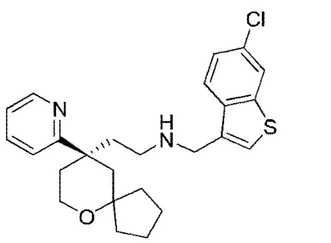, 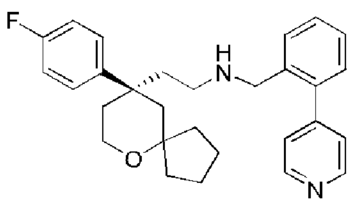,

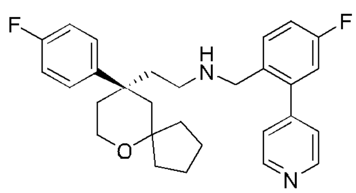, 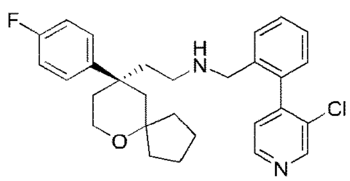, 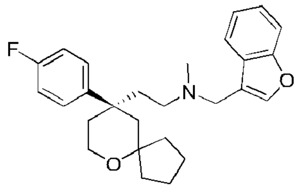,

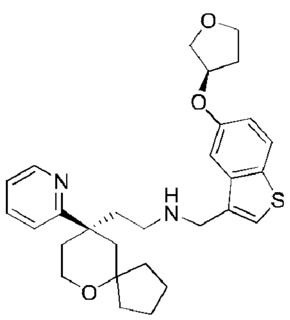, 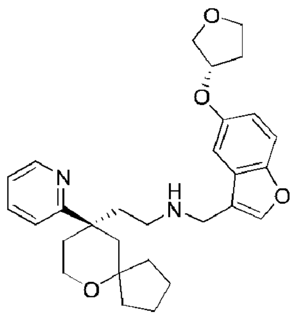, or 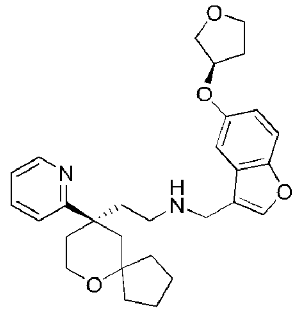.

**6.** The compound represented by formula (I) according to claim 5, wherein the compound is selected from:

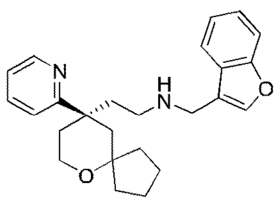, 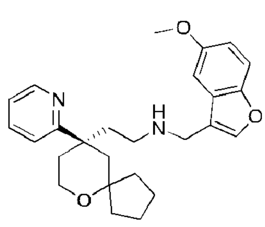, 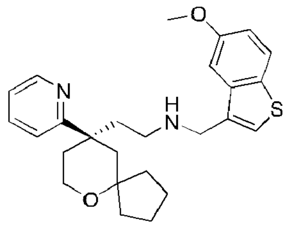,

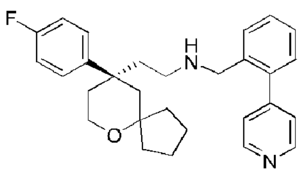, 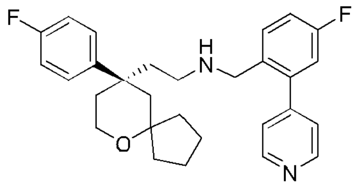, 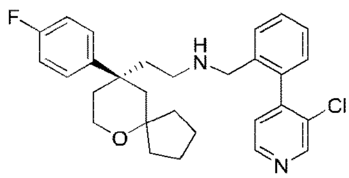,

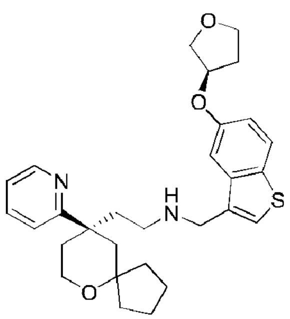, 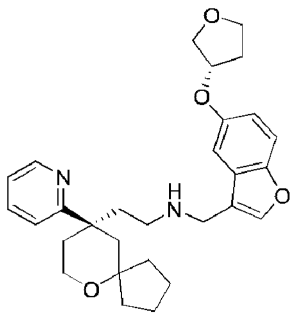, or 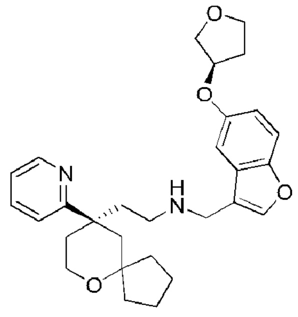.

**7.** A method of preparing the compound represented by formula (I) according to any one of claims 1 to 6, wherein, comprising the following step:

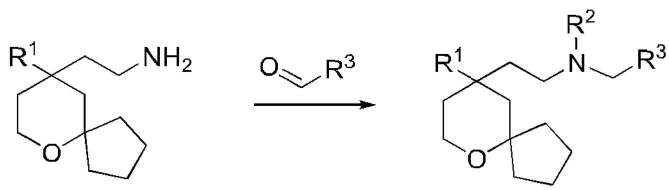

a I

wherein, $R^1$ , $R^2$, $R^3$ are as defined in any one of claims 1-6;
starting material a reacts with a raw material aldehyde to obtain target compound I with $R^2$=H; if $R^2$ is an alkyl group, the target compound I can be obtained after further alkylation reaction.

**8.** A method of preparing the compound represented by formula (la) according to claim 4, wherein, comprising the following step:

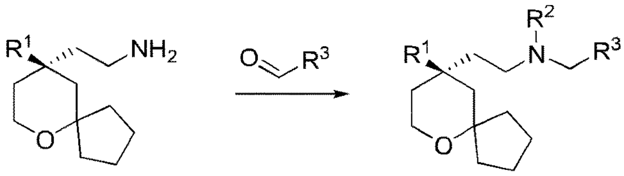

a' Ia

wherein $R^1$ is selected from

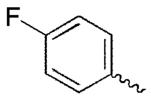, or 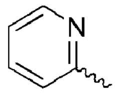;

$R^2$ is selected from a hydrogen atom; $R^3$ is selected from

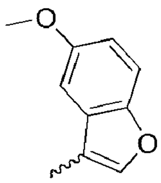, 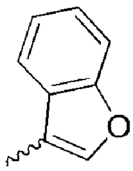, 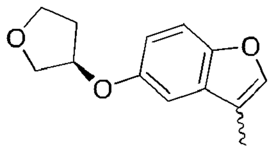, or 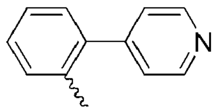;

starting material a' reacts with a raw material aldehyde to obtain target compound la.

**9.** Use of the compound represented by formula (I) according to claim 1 in the preparation of μ receptor agonist drug.

**10.** The use according to claim 9, wherein the use is one in the preparation of an analgesic drug.

**11.** The use according to claim 10, wherein the use is one in the preparation of a drug for moderate to severe pain.

## INTERNATIONAL SEARCH REPORT

International application No.
**PCT/CN2021/110702**

### A. CLASSIFICATION OF SUBJECT MATTER

C07D 405/14(2006.01)i； C07D 407/12(2006.01)i； C07D 409/14(2006.01)i； A61K 31/352(2006.01)i； A61K 31/4433(2006.01)i； A61K 31/4436(2006.01)i； A61P 29/00(2006.01)i

According to International Patent Classification (IPC) or to both national classification and IPC

### B. FIELDS SEARCHED

Minimum documentation searched (classification system followed by classification symbols)

C07D; A61K; A61P

Documentation searched other than minimum documentation to the extent that such documents are included in the fields searched

Electronic data base consulted during the international search (name of data base and, where practicable, search terms used)

CNABS, CNTXT, CNKI, 万方, WPI, EPODOC, STN-CAPLUS, STN-REGISTRY, 成都苑东, 阿片, 受体, 氧杂螺 4w 癸烷, 苯并噻吩, 苯并呋喃, 吡啶, 疼痛, opioid, receptor, MOR, OR, +oxaspiro+ 4w +decan+, +benzothiophene+, +benzofuran+, +pyridin+, pain, structural formula search

### C. DOCUMENTS CONSIDERED TO BE RELEVANT

| Category* | Citation of document, with indication, where appropriate, of the relevant passages | Relevant to claim No. |
|---|---|---|
| X | CN 103702561 A (TREVENA, INC.) 02 April 2014 (2014-04-02)<br>description paragraphs [0011]-[0032], [0104], [0232], [0311]-[0316], [0336]-[340] | 1-11 |
| X | CN 108601775 A (TREVENA, INC.) 28 September 2018 (2018-09-28)<br>description paragraphs [0025]-[0046], [0118], [0254], [0345]-[0350], [0388]-[0397], [0508]-[0509] | 1-11 |
| X | CN 108366997 A (TREVENA, INC.) 03 August 2018 (2018-08-03)<br>description paragraphs [0022]-[0043], [0075], [0220]-[0222], [0242]-[0244] | 1-6, 9-11 |
| X | CN 109206417 A (SICHUAN HAISCO PHARMACEUTICAL CO., LTD.) 15 January 2019 (2019-01-15)<br>description paragraphs [0015]-[0040], [0150]-[0152], [0220]-[0222], [0248], [1455]-[1477] | 1-11 |
| X | CN 109516982 A (SHANGHAI HUAHUITUO PHARMACEUTICAL TECHNOLOGY CO., LTD. et al.) 26 March 2019 (2019-03-26)<br>description paragraphs [0006]-[0052], [0082]-[0083], [0315]-[0334] | 1-11 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **11 October 2021** | **04 November 2021** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088**<br>**China** | |
| Facsimile No. **(86-10)62019451** | Telephone No. |

Form PCT/ISA/210 (second sheet) (January 2015)

**INTERNATIONAL SEARCH REPORT**
**Information on patent family members**

International application No.
**PCT/CN2021/110702**

| Patent document cited in search report | | | Publication date (day/month/year) | Patent family member(s) | | | Publication date (day/month/year) |
|---|---|---|---|---|---|---|---|
| CN | 103702561 | A | 02 April 2014 | EP | 2688403 | A1 | 29 January 2014 |
| | | | | KR | 20190072665 | A | 25 June 2019 |
| | | | | US | 10588898 | B2 | 17 March 2020 |
| | | | | JP | 2018048193 | A | 29 March 2018 |
| | | | | BR | 112013024136 | A2 | 14 January 2020 |
| | | | | HR | P20171021 | T1 | 22 September 2017 |
| | | | | JP | 2016155843 | A | 01 September 2016 |
| | | | | US | 2017326124 | A1 | 16 November 2017 |
| | | | | ME | 02754 | B | 20 January 2018 |
| | | | | AU | 2017200745 | A1 | 23 February 2017 |
| | | | | PL | 2688403 | T3 | 29 September 2017 |
| | | | | EP | 2688403 | A4 | 15 October 2014 |
| | | | | WO | 2012129495 | A1 | 27 September 2012 |
| | | | | EP | 3290415 | A1 | 07 March 2018 |
| | | | | US | 2013331408 | A1 | 12 December 2013 |
| | | | | IL | 243318 | D0 | 29 February 2016 |
| | | | | US | 8835488 | B2 | 16 September 2014 |
| | | | | US | 2012245181 | A1 | 27 September 2012 |
| | | | | US | 9849119 | B2 | 26 December 2017 |
| | | | | JP | 6243949 | B2 | 06 December 2017 |
| | | | | DK | 2688403 | T3 | 24 July 2017 |
| | | | | US | 2019343819 | A1 | 14 November 2019 |
| | | | | HK | 1250984 | A1 | 18 January 2019 |
| | | | | NZ | 713143 | A | 26 May 2017 |
| | | | | AU | 2012230761 | A1 | 02 May 2013 |
| | | | | IL | 228506 | D0 | 31 December 2013 |
| | | | | EA | 201391332 | A1 | 30 April 2014 |
| | | | | US | 9044469 | B2 | 02 June 2015 |
| | | | | SI | 2688403 | T1 | 31 August 2017 |
| | | | | US | 9642842 | B2 | 09 May 2017 |
| | | | | CY | 1119057 | T1 | 10 January 2018 |
| | | | | ES | 2632009 | T3 | 07 September 2017 |
| | | | | EP | 2688403 | B1 | 31 May 2017 |
| | | | | JP | 6649341 | B2 | 19 February 2020 |
| | | | | EA | 025456 | B1 | 30 December 2016 |
| | | | | JP | 5912169 | B2 | 27 April 2016 |
| | | | | KR | 102129247 | B1 | 06 July 2020 |
| | | | | KR | 101991326 | B1 | 20 June 2019 |
| | | | | KR | 20180100453 | A | 10 September 2018 |
| | | | | CA | 2830742 | A1 | 27 September 2012 |
| | | | | HU | E032948 | T2 | 28 November 2017 |
| | | | | NZ | 615993 | A | 27 November 2015 |
| | | | | KR | 20140047599 | A | 22 April 2014 |
| | | | | KR | 101991327 | B1 | 20 June 2019 |
| | | | | IL | 228506 | A | 30 November 2016 |
| | | | | US | 2016250199 | A1 | 01 September 2016 |
| | | | | JP | 2014508811 | A | 10 April 2014 |
| | | | | LT | 2688403 | T | 25 July 2017 |
| | | | | AU | 2017200745 | B2 | 22 February 2018 |
| CN | 108601775 | A | 28 September 2018 | KR | 20180094938 | A | 24 August 2018 |

Form PCT/ISA/210 (patent family annex) (January 2015)

**INTERNATIONAL SEARCH REPORT**
**Information on patent family members**

International application No.
**PCT/CN2021/110702**

| Patent document cited in search report | Publication date (day/month/year) | Patent family member(s) | Publication date (day/month/year) |
|---|---|---|---|
| | | CA 3008637 A1 | 22 June 2017 |
| | | JP 2019503351 A | 07 February 2019 |
| | | IL 260003 D0 | 31 July 2018 |
| | | EP 3389656 A4 | 19 June 2019 |
| | | AU 2016370860 A1 | 12 July 2018 |
| | | EP 3389656 A1 | 24 October 2018 |
| | | WO 2017106547 A1 | 22 June 2017 |
| | | HK 1254673 A1 | 26 July 2019 |
| | | US 2019008803 A1 | 10 January 2019 |
| CN 108366997 A | 03 August 2018 | CA 3007020 A1 | 22 June 2017 |
| | | US 2017333385 A1 | 23 November 2017 |
| | | IL 259992 A | 25 March 2021 |
| | | JP 2019504113 A | 14 February 2019 |
| | | EP 3389657 A4 | 07 August 2019 |
| | | WO 2017106306 A1 | 22 June 2017 |
| | | US 10588889 B2 | 17 March 2020 |
| | | IL 259992 D0 | 31 July 2018 |
| | | AU 2016372028 A1 | 12 July 2018 |
| | | EP 3389657 A1 | 24 October 2018 |
| | | IL 281293 D0 | 29 April 2021 |
| | | HK 1254672 A1 | 26 July 2019 |
| | | HK 1251472 A1 | 01 February 2019 |
| | | KR 20180093930 A | 22 August 2018 |
| | | US 2021023043 A1 | 28 January 2021 |
| CN 109206417 A | 15 January 2019 | None | |
| CN 109516982 A | 26 March 2019 | WO 2019052557 A1 | 21 March 2019 |
| | | JP 2020534294 A | 26 November 2020 |
| | | EP 3686198 A4 | 21 April 2021 |
| | | US 2020270230 A1 | 27 August 2020 |
| | | EP 3686198 A1 | 29 July 2020 |

Form PCT/ISA/210 (patent family annex) (January 2015)